# FASCICULE DE BREVET EUROPEEN

(11) **EP 3 325 033 B1**
(45) Date de publication et mention de la délivrance du brevet: **12.01.2022**
(21) Numéro de dépôt: 16747838.7
(22) Date de dépôt: 15.07.2016
(51) Int. Cl.: A61L 27/56, A61L 27/12, F26B 5/06, C04B 30/00, B01J 3/06, C04B 38/06, C01B 25/32

(54) **PROCEDE DE FABRICATION D'UN MATERIAU MONOLITHIQUE POREUX**
VERFAHREN ZUR HERSTELLUNG EINES PORÖSEN MONOLITHISCHEN MATERIALS
METHOD FOR PRODUCING A POROUS MONOLITHIC MATERIAL

(30) Priorité: 17.07.2015 FR 1556771
(43) Date de publication de la demande: 30.05.2018
(73) Titulaire: Centre National de la Recherche Scientifique, 75016 Paris (FR); Université de Bordeaux, 33000 Bordeaux (FR)
(72) Inventeur: LARGETEAU, Alain, 33610 Cestas (FR); PRAKASAM, Mythili, 33400 Talence (FR)
(74) Mandataire: IPAZ
(86) Numéro de dépôt international: PCT/FR2016/051821
(87) Numéro de publication internationale: WO 2017/013339

(56) Documents cités:
- EP-A1- 1 500 405
- EP-A1- 1 566 186
- EP-A1- 1 964 583
- CN-A- 103 599 561
- JP-A- 2000 189 510
- JP-A- 2009 254 547
- US-A1- 2014 141 224

## Description

La présente invention concerne un procédé de fabrication d'un matériau monolithique poreux à partir d'au moins une poudre, de préférence minérale, ce procédé comprenant au moins une étape de compression à basse température d'un mélange à base de poudre minérale et d'au moins un solvant, préférentiellement de l'eau. Les matériaux obtenus par ce procédé présentent des propriétés mécaniques améliorées par rapport aux matériaux de l'art antérieur. Dans le cas des matériaux à application médicale, comme l'hydroxyapatite par exemple, ils présentent également une biocompatibilité améliorée par rapport aux matériaux de l'art antérieur.

### Etat de la technique antérieure

La fabrication de matériaux monolithiques poreux à base de métaux alcalins ou alcalino-terreux, comme par exemple l'hydroxyapatite, le dioxyde de titane, l'alumine ou la zircone, peut être réalisée à l'heure actuelle au moyen de différents procédés :
On peut partir d'une poudre minérale, à laquelle est appliquée une technique de frittage, qui consiste en un chauffage à haute température, pour rigidifier la structure granulaire de départ. Cette technique, qui inclut différentes variantes (frittage conventionnel ou frittage libre, frittage par pression à chaud, frittage par pression isostatique à chaud) n'est pas dépourvue d'inconvénients. Le chauffage à haute température entraine une modification de la structure chimique de la matière première minérale : élimination partielle des atomes d'oxygène et des hydroxydes notamment. Une hydroxyapatite soumise à un tel traitement présente une biocompatibilité inférieure à l'hydroxyapatite de départ. Un dioxyde de titane chauffé au delà de 500°C n'est plus biocompatible, car la forme anatase, seule biocompatible, est dégradée à cette température. L'alumine Al₂O₃ et la zircone ZrO₂ présentent également des transitions structurales à température élevée et peuvent perdre leur biocompatibilité par application d'un tel traitement. Un autre inconvénient de cette méthode est que l'on ne peut pas incorporer de matière première organique à la poudre minérale avant le frittage, car elle serait décomposée par ce traitement thermique. On ne peut donc incorporer de matière première organique, telle que des principes actifs de médicament, qu'après la formation du monolithe, ce qui augmente le nombre d'étapes et rend plus difficile l'obtention d'un matériau homogène.

Il existe d'autres techniques que le frittage pour la fabrication de matériaux monolithiques poreux à base de matière première minérale : La fabrication additive (impression tridimensionnelle) est une technique qui nécessite un chauffage localisé par un laser, ce qui revient à un chauffage à haute température. C'est notamment le cas dans les procédés de fusion sélective par laser et de frittage sélectif par laser. On peut également procéder au coulage dans un moule d'une barbotine contenant la matière première minérale et un additif chimique servant de porogène pour la formation des pores. Ensuite, soit le porogène est éliminé sous forme de gaz par chauffage ou bien il reste dans le matériau, s'il n'y a pas de chauffage, sous forme «d'éponge». La structure formée par cet additif donne au matériau une tenue mécanique souple mais pas rigide. On sait également fabriquer des monolithes poreux en procédant par coulage dans un moule froid (à température négative) d'une barbotine contenant de l'eau. L'eau contenue dans la barbotine forme des cristaux de glace qui sont ensuite éliminés, par lyophilisation par exemple. Un tel procédé donne un matériau très friable. En règle générale il est nécessaire d'ajouter un gélifiant pour donner de la tenue mécanique à un tel matériau, et sa résistance reste néanmoins assez faible. Il peut même être fritté ultérieurement pour améliorer sa résistance. Il peut arriver que certains éléments de la barbotine restant dans le matériau final ne soient pas biocompatibles.

Il est connu de soumettre une poudre à une pression isostatique à température ambiante de façon à former un monolithe. On obtient alors un matériau dense mais non poreux.

La demande KR20110088903 décrit un procédé de fabrication d'un matériau poreux à base d'hydroxyapatite, ce procédé comprenant la formation d'une barbotine à base d'alcool qui est congelée, l'élimination de la solution alcoolique porogène puis le frittage du matériau. Les inconvénients de ce procédé sont notamment l'utilisation d'une étape de frittage qui modifie la structure chimique du matériau.

La demande CN103599561 décrit un matériau composite, utilisable comme substitut osseux, à base de magnésium et d'hydroxyapatite, le procédé de fabrication de ce matériau comprenant le mélange puis le pressage à froid du mélange dans un moule et le frittage du matériau.

La demande LV14492 décrit un matériau poreux à base d'hydroxyapatite doté d'une distribution de pores bimodale, ce matériau pouvant être utilisé comme matériau de substitution osseuse. Le procédé de fabrication met en œuvre du bicarbonate d'ammonium comme agent porogène. Un tel matériau présente des propriétés mécaniques peu satisfaisantes.

La demande US2014/0314824 décrit un procédé de fabrication d'un support poreux biocompatible utilisable pour la culture de tissus, à base de chitosane, hydroxyapatite et amylopectine. La fabrication du matériau comporte la dissolution du chitosane dans une solution d'acide acétique, l'addition d'hydroxyapatite puis d'amylopectine, enfin la congélation de la solution, sa neutralisation par une solution aqueuse basique, le lavage et le séchage du matériau. Ce procédé fournit un matériau dont la tenue mécanique n'est pas totalement satisfaisante.

Le document JP2009-254547 décrit un procédé de fabrication d'un matériau de régénération osseuse à partir d'un phosphate octocalcique, précurseur de l'hydroxyapatite. Ce matériau est mélangé avec un polymère hydrosoluble ou un polysaccharide tel que de l'alginate de sodium et l'ensemble est soumis à un traitement consistant en une élévation de pression allant de 4,1.10⁻³MPa à 3,7.10⁻²MPa. Un traitement thermique est nécessaire pour stériliser le matériau.

Le document US2014/0141224 décrit un matériau de type mousse de carbone préparé à partir d'un matériau carboné, tel que des nanotubes de carbone, qui est dissout en milieu acide, la solution est moulée et la source de carbone est coagulée. Le matériau coagulé peut être lyophilisé ou séché.

Le document EP 1 566 186 décrit un matériau poreux apatite/collagène coagulé. Ce matériau est préparé à partir d'un mélange d'un composite apatite/collagène et de collagène. Ce mélange est gélifié puis lyophilisé et réticulé.

Le document EP 1 964 583 décrit un procédé de production d'un matériau composite collagène/hydroxyapatite, comprenant la formation d'une suspension homogène de collagène et d'hydroxyapatite dans une solution acide, la lyophilisation de la solution et éventuellement la réticulation du matériau.

Le document EP 1 500 405 décrit un procédé de production de matériaux composites poreux à partir d'un sel de calcium et de collagène, comprenant la congélation d'un complexe formé d'un sel de calcium et de collagène au moins en partie gélifié, et sa lyophilisation.

Aucun de ces procédés ne conduit à un matériau présentant à la fois une porosité et une tenue mécanique satisfaisantes.

L'objectif de l'invention a été de surmonter les problèmes rencontrés avec les matériaux de l'art antérieur. En particulier, on a cherché à mettre au point un procédé fiable et reproductible qui permette, à partir d'une poudre, en particulier une poudre minérale, choisie, de former un monolithe de même composition chimique, sans dégradation de la structure chimique de départ, ce matériau monolithique étant doté de bonnes propriétés de résistance mécanique. On a également cherché à mettre au point un procédé qui permette d'incorporer d'éventuels composants de nature organique dans le matériau dès les premières étapes de sa fabrication, de façon à ce que le matériau monolithique présente une composition homogène. On a donc cherché à mettre au point un procédé de fabrication qui ne dégrade pas les molécules organiques, notamment qui ne comporte pas d'étape de chauffage à des températures élevées, telle qu'une étape de frittage par exemple.

### Résumé de l'invention

L'invention concerne un procédé de fabrication d'un matériau monolithique poreux, ce procédé comprenant au moins les étapes suivantes :
(a) Fourniture d'au moins une poudre minérale ou organique,
(b) Préparation d'un mélange comprenant au moins la poudre de l'étape (a) et au moins un solvant, de préférence de l'eau,
(c) Conditionnement du mélange de l'étape (b) dans un emballage étanche en matériau élastiquement déformable pour former un échantillon,
(d) Application à l'échantillon de l'étape (c) d'une pression supérieure ou égale à 50MPa à une température où le solvant est sous forme solide,
(e) Retour à la pression atmosphérique,
(f) Ouverture de l'emballage et récupération du matériau,
(g) Elimination du solvant à une température où le solvant est sous forme solide.

Selon un mode de réalisation préféré, la poudre est choisie parmi les poudres minérales ou inorganiques et de préférence parmi : les métaux, notamment les métaux alcalins et alcalino-terreux, les métaux de transition, leurs sels et leurs dérivés, notamment les oxydes, les hydroxydes, les sels de phosphate, de carbonate, les borures, les carbures, les nitrures ; les céramiques et les matériaux composites.

Selon un mode de réalisation préféré, la poudre est minérale ou inorganique et est choisie parmi : l'hydroxyapatite, l'alumine, le dioxyde de titane, la zircone, la zircone stabilisée à l'Yttrium (YSZ), la zircone stabilisée à la Magnésie (PSZ).

Selon un mode de réalisation préféré, le mélange de l'étape (b) comprend au moins un composant organique, de préférence choisi parmi : un principe actif thérapeutique ; des peptides ; des protéines ; des agents coagulants ; des marqueurs ; des facteurs de croissance ; des polymères naturels ou de synthèse ; des agents de dopage pour l'électronique ; des polymères électroconducteurs ; des capteurs chimiques ; un agent de réticulation ; un solvant miscible à l'eau.

Selon un mode de réalisation préféré, le mélange comprend de 50 à 95 % en masse de poudre, préférentiellement de poudre minérale ou inorganique, par rapport à la masse totale du mélange.

Selon un mode de réalisation préféré, le mélange comprend de 60 à 90 % en masse de poudre, préférentiellement de poudre minérale ou inorganique, par rapport à la masse totale du mélange.

Selon un mode de réalisation préféré, la pression appliquée à l'étape (d) est une pression isostatique.

Selon un mode de réalisation préféré, l'étape (d) comprend au moins les sous-étapes suivantes :
(d1) Un conditionnement de l'échantillon à pression atmosphérique et à une température choisie pour que le solvant, de préférence l'eau, soit sous forme solide,
(d2) L'application d'une pression supérieure ou égale à 50MPa à une température choisie pour que le solvant, de préférence l'eau, soit sous forme solide.

Selon un mode de réalisation préféré, l'étape (d) comprend au moins un conditionnement à une température allant de -5°C à -60°C et à une pression allant de 50MPa à 700MPa.

Selon un mode de réalisation préféré, l'étape (d) comprend au moins un conditionnement à une température allant de -5°C à -60°C et à une pression allant de 100MPa à 600MPa.

Selon un mode de réalisation préféré, le solvant est de l'eau.

Selon un mode de réalisation préféré, l'élimination de l'eau à l'étape (g) est réalisée par lyophilisation.

Selon un mode de réalisation préféré, le matériau de l'invention est à base d'au moins un matériau choisi parmi les métaux, les sels de métaux, les dérivés de métaux, les céramiques et les matériaux composites, de préférence à base d'un matériau choisi parmi l'hydroxyapatite, l'alumine, la zircone, TiO₂.

Le procédé de l'invention permet, à partir d'un matériau de nature minérale ou organique sous forme de poudre, d'obtenir un monolithe présentant une structure et une composition chimique inchangées par rapport à la poudre de départ, mais une cohésion et une résistance mécanique élevées.

Le procédé de l'invention permet d'obtenir un matériau monolithique poreux présentant une porosité sensiblement homogène.

Le procédé de l'invention permet d'obtenir des matériaux monolithiques à base d'un matériau de nature minérale et comprenant des composants organiques dispersés de façon homogène.

Le procédé de l'invention permet d'obtenir facilement un matériau monolithique de la forme souhaitée.

Le procédé de l'invention est simple à mettre en œuvre et reproductible.

Le procédé de l'invention permet d'éviter le développement d'une contamination microbienne, notamment bactérienne, au cours de la fabrication.

Les propriétés du matériau monolithique (porosité, résistance mécanique) peuvent être modulées grâce au contrôle des paramètres du procédé (quantité de solvant, en particulier d'eau, pression, température, durée d'application de la pression).

Le matériau monolithique poreux obtenu par le procédé de l'invention se distingue des matériaux de l'art antérieur par : sa structure et sa composition chimique inchangées par rapport au matériau de départ, sa résistance mécanique, sa porosité sensiblement homogène. En particulier, la porosité est répartie de façon sensiblement uniforme sur le volume du matériau et la taille des pores est sensiblement homogène sur l'ensemble du matériau.

### Figures :

- Figure 1 : Représentation schématique des étapes du procédé selon l'invention
- Figure 2 : Diagramme de phase de l'eau

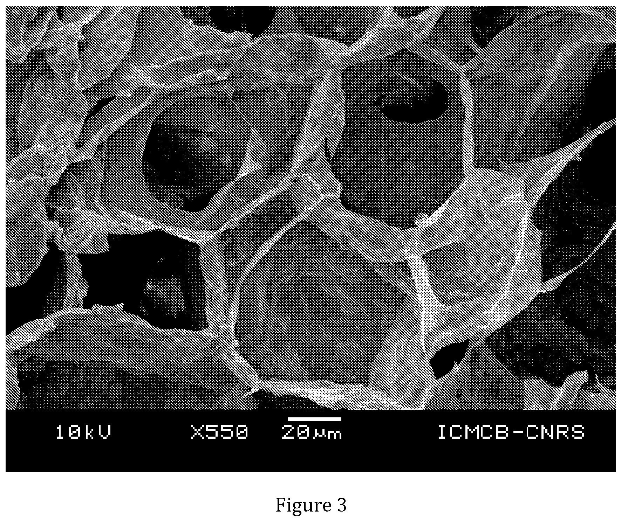

- Figure 3 : Microstructure d'un matériau obtenu par compression isostatique à froid, à base de HAp, de gélatine et de 40% d'eau en masse, visualisée par microscopie électronique à balayage (MEB)

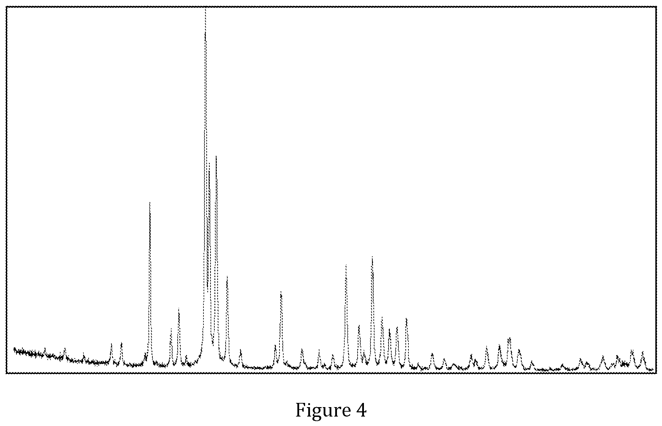

- Figure 4 : Diffractogramme aux rayons X du matériau à base de HAp, de gélatine et de 40% d'eau, obtenu par compression isostatique à froid.

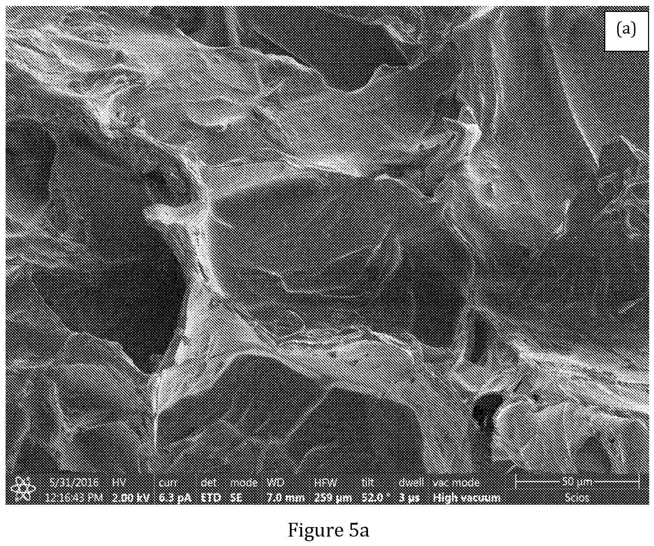

- Figure 5a : Diffractogramme aux rayons X du matériau à base de 80% HAp, et de 20% d'eau (exemple 1.1b), obtenu par compression isostatique à froid.

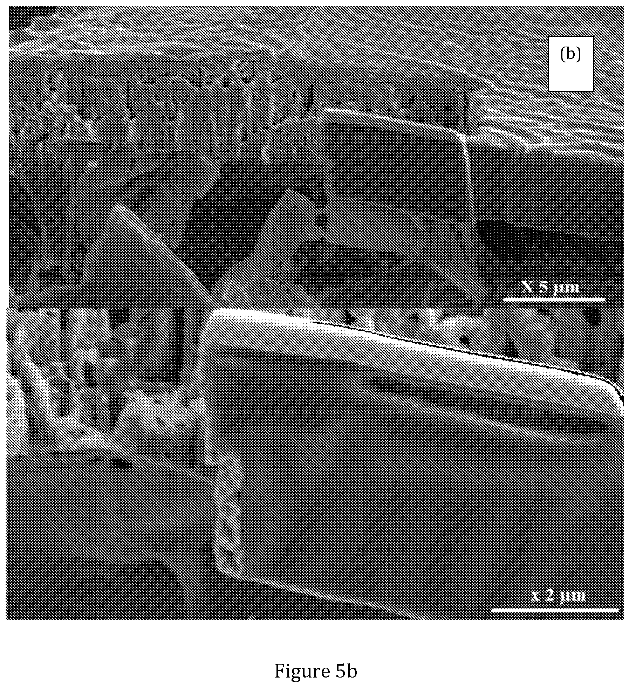

- Figure 5b : Cliché de micro-tomographie aux rayons X du matériau à base de 80% HAp, et de 20% d'eau (exemple 1.1b), obtenu par compression isostatique à froid.

### Description détaillée

L'invention concerne un procédé de fabrication d'un matériau monolithique poreux, à partir d'au moins une poudre minérale ou organique, ce procédé comprenant la formation d'un mélange comprenant au moins la poudre et au moins un solvant, préférentiellement de l'eau pour les applications requérant une biocompatibilité, et éventuellement des composants organiques. Le mélange est placé dans un emballage en plastique scellé et refroidi de façon à ce que le solvant présent dans le mélange forme des cristaux, notamment de façon à ce que l'eau utilisée comme solvant forme des cristaux de glace. Le matériau congelé est alors soumis à une pression élevée de façon à consolider la structure et former un monolithe à partir des grains de poudre, en particulier de poudre minérale. Après retour à une pression atmosphérique et à température ambiante, l'emballage est ouvert et le matériau est récupéré puis le solvant, notamment l'eau, est éliminé dans des conditions permettant d'éviter de dégrader la structure chimique du monolithe et/ou les composants organiques éventuels. En particulier, le solvant est préférentiellement éliminé à une température à laquelle il est sous forme solide.

Le procédé de l'invention consiste à consolider un échantillon, qui est un mélange contenant de la poudre, par exemple de l'hydroxyapatite (HAp) en présence d'une certaine proportion de solvant, notamment d'eau, sous forme de cristaux. La densification se fait en milieu isostatique et avec une pression pouvant atteindre jusqu'à 800MPa transmise par un fluide transmetteur de pression au travers de l'emballage souple contenant l'échantillon. L'emballage permet de manipuler aisément l'échantillon et d'éviter la contamination par le fluide transmetteur de pression

### La poudre minérale ou organique :

Le procédé de l'invention peut être mis en œuvre à partir de tout matériau disponible sous forme de poudre, et en particulier à partir d'une poudre d'un matériau minéral ou organique. La poudre du matériau de départ doit être insoluble dans le solvant, notamment dans l'eau lorsqu'elle est utilisée comme solvant. On peut citer comme exemple de matériau utilisable : les métaux, notamment les métaux alcalins et alcalino-terreux, les métaux de transition, leurs sels et leurs dérivés, notamment les oxydes, les hydroxydes, les sels de phosphate, de carbonate, les borures, les carbures, les nitrures, on peut citer également les céramiques et les matériaux composites. On peut également citer les poudres de carbone. Parmi les poudres organiques susceptibles d'être mises en œuvre dans le procédé de l'invention, on peut citer notamment les poudres de polymères.

De façon avantageuse, le procédé de l'invention s'applique à des poudres minérales ou inorganiques.

Le procédé trouve un intérêt particulier pour la fabrication de monolithes à partir de matières premières telles que :
- les phosphates de calcium, notamment l'hydroxyapatite (HA) : Ca₁₀(PO4)₆(OH)₂, le phosphate tricalcique bêta (β-TCP) : Ca₃(PO₄)₂, les phosphates de calcium biphasiques (BCP) résultant du mélange de HA et de β-TCP, l'oxyapatite, le tétracalcium phosphate, le pyrophosphate de calcium, la fluoroapatite ; parmi ces composés, les préférés sont l'hydroxyapatite et le BCP qui favorisent particulièrement la reconstruction osseuse ;
- les céramiques, notamment l'alumine (Al₂O₃), la zircone (ZrO2), la Zircone stabilisée à l'Yttrium (YSZ) ou à la Magnésie (PSZ), la magnésie (MgO), le nitrure de bore (BN), le nitrure de silicium (Si₃N₄), le carbure de silicium (SiC), le titanate d'aluminium (Al₂TiO₅), le nitrure d'aluminium (AIN) ;
- les dérivés du titane notamment le dioxyde de titane ;
- le quartz ou dioxyde de silicium (SiO₂), comprenant éventuellement des traces d'éléments tels que Al, Li, B, Fe, Mg, Ca, Ti, Rb, Na, OH ;

La poudre mise en œuvre dans le procédé comme matériau de départ peut être composée d'un seul matériau (par exemple hydroxyapatite) ou elle peut être composée d'un mélange de matériaux (par exemple BCP).

La poudre de matériau de départ présente de façon préférentielle une taille de grains inférieure ou égale à 1 µm. De préférence elle présente une taille de grains allant de 0,1 à 500 nm, avantageusement de 0,5 à 100 nm.

### Le mélange de poudre et de solvant :

Le solvant peut être tout solvant capable de solidifier dans des conditions de pression et de température choisies. Avantageusement, le solvant est de l'eau, dont on maîtrise facilement les conditions de formation de cristaux de glace et les conditions d'évaporation. Par ailleurs l'eau présente l'avantage d'être compatible avec les applications à des biomatériaux.

La poudre, en particulier la poudre minérale ou inorganique, ne doit pas être soluble dans le solvant, en particulier dans l'eau.

Le mélange de poudre et de solvant, en particulier d'eau, doit comporter suffisamment de solvant pour former, après refroidissement du mélange, un réseau de cristaux, notamment de cristaux de glace, qui est à l'origine de la porosité dans le matériau monolithique. Le contrôle de la quantité de solvant, en particulier la quantité d'eau, permet de contrôler la morphologie du matériau final, notamment la quantité de solvant influe sur le volume poreux du matériau final. Il faut que le mélange comprenne suffisamment de poudre pour former une pâte homogène. Une trop grande quantité de solvant, en particulier d'eau, ne permet pas la formation d'un matériau monolithique car les grains sont trop espacés pour permettre cette formation. La quantité de poudre incorporée au mélange dépend également de la densité de la poudre.

Le mélange de poudre et de solvant, en particulier d'eau, comporte avantageusement de 50 à 95%, encore mieux de 60 à 90%, en masse de poudre par rapport à la masse totale du mélange.

Le mélange de poudre et de solvant, en particulier d'eau, peut également comprendre d'autres composants, organiques ou inorganiques, comme par exemple :
- dans le cas des matériaux de substitution osseuse : des principes actifs thérapeutiques ; des peptides ; des protéines ; des agents coagulants, comme par exemple de la thrombine ou du chlorure de calcium ; des marqueurs, notamment des marqueurs de fluorescence ; des facteurs de croissance ; des polymères naturels ou de synthèse comme par exemple de la gélatine, du collagène, de l'acide hyaluronique, du chitosane, de la kératine, de l'alginate, de la fibronectine, du fibrinogène, de l'acide polyglycolique (PGA), de l'acide polylactique (PLA), des copolymères des acides lactique et glycolique (PLGA) ;
- dans le cas des matériaux pour application dans l'électronique : des agents de dopage ; des polymères électroconducteurs ;
- dans le cas des matériaux pour fabriquer des capteurs chimiques : des molécules susceptibles de réagir avec la molécule cible ;
- un agent de réticulation qui permet de consolider la structure du matériau par la création d'une réticulation chimique, comme par exemple du soufre, un diacide, un anhydride, une polyamine ;
- des solvants miscibles à l'eau, dès lors qu'ils n'empêchent pas la solidification de l'eau à basse température.

Lorsqu'un matériau additionnel, organique ou minéral, est présent dans le mélange, il représente avantageusement jusqu'à 10% en masse par rapport à la masse totale du mélange.

De préférence, on met en œuvre des matières premières additionnelles, organiques ou minérales, solubles dans le solvant choisi, de façon à faciliter l'obtention d'un matériau homogène. Lorsque, de façon préférentielle, le solvant est de l'eau, on choisit des matières premières solubles dans l'eau.

### L'emballage du matériau de départ

Le procédé de l'invention repose sur le choix d'un matériau d'emballage qui peut être refermé de façon étanche. Pour cela on choisit avantageusement un matériau thermoscellable tel qu'un thermoplastique. Le matériau constitutif de l'emballage doit être élastiquement déformable, notamment il doit être élastiquement déformable à des températures très basses (négatives) telles que des températures aux environs de -40°C à -60°C.

Parmi les matériaux d'emballage susceptibles d'être mis en œuvre dans le procédé de l'invention, on peut citer notamment le polyéthylène (PE) ou les silicones. On peut également mettre en œuvre tout autre film composé d'une ou de plusieurs couches de matériau de nature chimique différente ou non capable d'être thermoscellé et élastiquement déformable aux températures et pressions de mise en œuvre du procédé. La forme de l'emballage permet de contrôler la forme du matériau final que l'on souhaite obtenir.

### Les étapes du procédé :

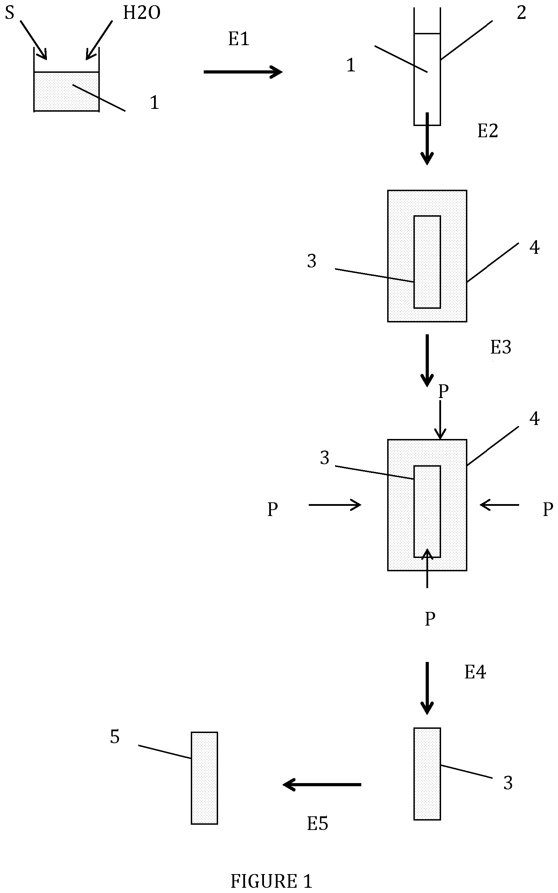

Le procédé de l'invention est illustré de façon schématique par la figure 1 à laquelle il sera fait référence :
La poudre solide (S) et l'eau, ainsi que d'éventuels additifs (non représentés) sont préparées de façon à former un mélange homogène 1. Le mélange 1 est placé dans une poche 2 en matériau thermoscellable et élastiquement déformable (étape E1). Le mélange 1 conditionné dans son emballage étanche de façon à former un échantillon 3 est placé dans un bain thermostaté à température négative 4 à pression atmosphérique (étape E2 facultative). L'échantillon 3 est ensuite soumis à une élévation de pression P dans des conditions isostatiques et à une température à laquelle l'eau, comme solvant, contenue dans le mélange de départ est solide. Les conditions isostatiques sont obtenues en plaçant l'échantillon 3 emballé dans un milieu transmetteur de pression (milieu cryogénique 4), en particulier un fluide transmetteur de pression, comme par exemple de l'éthylène glycol ou une huile de silicone. L'échantillon 3 est ensuite replacé en conditions de température et pression ambiante (étape E4). Le matériau polymère d'emballage est ôté, le matériau est ensuite lyophilisé (étape E5) de façon à éliminer l'eau rapidement et à obtenir le matériau déshydraté et consolidé 5.

Avantageusement, l'échantillon est soumis à une pression supérieure ou égale à 100MPa pendant au moins 5 min.

L'originalité de ce procédé réside dans l'application d'une pression isostatique P lorsque l'échantillon contenu dans son emballage est plongé dans le milieu transmetteur de pression, qui est à température négative suffisamment faible (jusqu'à - 40°C ou -60°C) pour transformer l'eau, contenue dans l'échantillon, en cristaux de glace. Ces cristaux de glace se transforment en un réseau solide (Template) servant de support autour duquel les particules constituant la poudre (HAp par exemple) sont comprimées.

Il est également possible de modifier la morphologie des cristaux de glace formés dans le tube à l'aide d'un gradient de température extérieure, à la température cryogénique, puis en appliquant de nouveau une pression supplémentaire à la haute pression, ce qui contribue à l'amélioration de la résistance mécanique. De plus la morphologie du cristal de glace peut être modifiée à l'aide du domaine de pression utilisé.

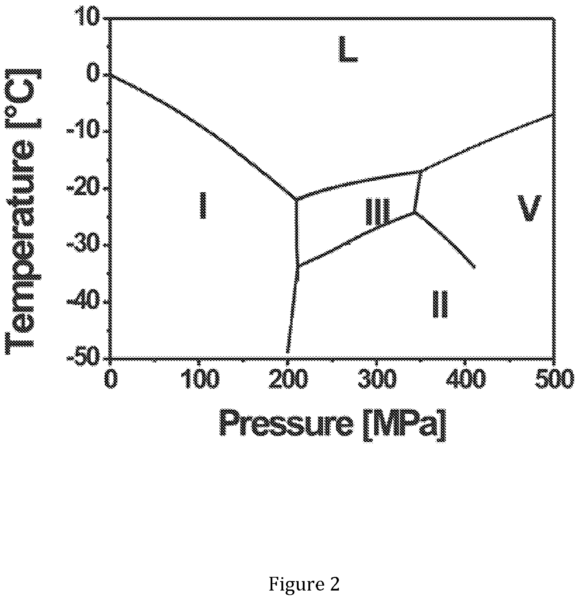

L'échantillon peut être soumis à plusieurs étapes successives d'application d'une pression isostatique, en faisant varier les paramètres de température et de pression en fonction du diagramme de phases de l'eau, de telle sorte que l'eau qu'il contient est solide. Une particularité du diagramme de phase de l'eau montre qu'il est possible de décongeler dans un domaine pouvant atteindre -20°C à 200 MPa, Ce domaine est à éviter lorsque les cristaux de glace sont formés. La formation des cristaux est possible par exemple en maintenant la poche quelques minutes dans le bain refroidi puis en poursuivant par l'application de pression ou bien par exemple en appliquant la pression dès que la poche est plongée dans le bain ou bien encore il est possible de démarrer le cycle depuis le domaine liquide sous pression. Il est ainsi possible au moyen des paramètres P, T, et t de faire suivre différents chemins dans le diagramme de phase pour faire varier la taille des cristaux de glace. Le diagramme de phase de l'eau (figure 2) montre l'existence de plusieurs variétés cristallographiques des cristaux de glace, différenciés par leur densité. Seule la glace I est moins dense que l'eau, toutes les autres variétés sont plus denses que celle de type I.

L'élévation de pression peut être obtenue par exemple en plaçant l'échantillon dans une enceinte elle-même placée dans un milieu cryogénique.

Lorsque la pression est supprimée puis que l'échantillon est retiré du bain toujours à température négative très faible, cet échantillon revient à température ambiante, les cristaux de glace reviennent sous forme liquide, sinon l'échantillon est conservé dans un congélateur avant d'être lyophilisé. Après ouverture de l'emballage, l'eau est retirée de façon à obtenir un produit déshydraté. De préférence, cette déshydratation est réalisée au moyen d'une lyophilisation (sous vide), ce qui permet la transformation de l'eau de l'état solide à l'état de vapeur sans passer par la phase liquide, soit par sublimation de préférence à la fusion. Ainsi les matières solubles dans l'eau, telles que les principes actifs thérapeutiques, ne sont pas affectées par cette étape, l'élimination de l'eau est rapide et efficace, on évite une éventuelle contamination bactérienne du matériau.

Après l'élimination d'eau, sous l'effet de la compression des particules autour du réseau solide (Template) de cristaux de glace, on obtient une structure consolidée de type monolithe poreux. Ce Template à base d'eau cristallisée sous forme de glace agit comme un porogène propre et naturel.

La pièce récupérée est donc rigide et présente une microstructure poreuse dont la taille des pores dépend de la taille des cristaux de glace préalablement formés. La proportion de pores en volume dans la pièce finale dépend du rapport volumique entre la phase liquide (eau) et la phase solide. La structure poreuse est continue, ce qui permet ensuite l'élimination de la phase aqueuse.

La taille et forme du cristal de glace dépendent du cycle thermique sous pression (température, temps, pression) parcouru dans le diagramme de phase (P, T) de l'eau. La taille du cristal de glace dépend du temps de présence dans le bain à température négative avant la montée en pression car plus ce temps est important et plus la croissance des cristaux est importante.

Selon un premier mode de réalisation, dans une première étape on conditionne l'échantillon placé dans son emballage à une température négative et à pression atmosphérique de telle sorte que l'eau contenue dans l'échantillon solidifie. Ensuite, selon ce mode de réalisation, on place l'échantillon congelé dans une presse isostatique, toujours à une température négative, la température étant adaptée en fonction du choix de la pression, pour que l'eau soit toujours sous forme solide.

Selon un second mode de réalisation, on soumet l'échantillon placé dans son emballage à une élévation de pression et à une température négative telle que l'eau reste liquide. Puis on augmente la pression et/ou on baisse la température de façon à former des cristaux de glace.

Cette possibilité donnée par le diagramme de phase repose sur le fait que l'eau est sous forme liquide à température négative (par exemple à -20°C) sous pression élevée (par exemple 200MPa). Cette particularité permet de supprimer toute germination/croissance des cristaux de glace dans l'échantillon. Il est ainsi possible de favoriser une germination foisonnante et donc très fine de cristaux de glace qui peut ensuite être utilisée pour obtenir une pièce consolidée à structure poreuse très fine dès que le couple température/pression est modifié par rapport au couple (T/P) de départ (par exemple -20°C/200MPa).

Il est à noter que la pression isostatique permet d'obtenir un monolithe présentant une structure poreuse équiaxe, donc sans orientation particulière. Mais il est possible d'avoir une structure orientée dans le cas d'un gradient thermique appliqué pendant la formation des cristaux de glace.

Les paramètres du procédé sont ajustés, comme la température du bain constituant le milieu transmetteur de pression, le temps de palier avant application de la pression, la vitesse de compression, le niveau de pression appliquée, la durée d'application de la pression, la vitesse de décompression, de telle façon que la densification soit suffisante pour obtenir une pièce présentant la consolidation souhaitée. La dureté du matériau est un facteur à prendre en considération : plus le matériau granulaire de départ est dur, plus il est nécessaire d'appliquer une pression forte pour former une structure monolithique.

De préférence, l'étape (d) du procédé comprend au moins un conditionnement à une température allant de -5°C à -60°C et à une pression allant de 50MPa à 700MPa, de préférence de 100MPa à 600MPa.

### Matériau monolithique poreux :

Le matériau monolithique poreux obtenu par le procédé de l'invention présente des propriétés innovantes par rapport aux matériaux de l'art antérieur :
La structure cristallographique et la composition chimiques sont inchangées par rapport au matériau minéral ou inorganique de départ du fait qu'il n'y a pas de chauffage, la résistance mécanique et la rigidité sont significativement supérieures comparativement au même matériau non chauffé, la rigidité est plus importante, la porosité du matériau est au moins aussi homogène que celle obtenue par les procédés de frittage ou les procédés par voie liquide utilisant un porogène.

La diffraction aux rayons X permet d'observer la conservation de la structure cristallographique.

La microscopie électronique permet d'observer la morphologie de la porosité.

La dureté est évaluée par un indenteur.

Le monolithe poreux obtenu par le procédé de l'invention est à base d'au moins un matériau choisi parmi les métaux, les dérivés de métaux, les oxydes de métaux, les hydroxydes de métaux, les céramiques, les composites, dont la structure chimique est celle des métaux, des dérivés de métaux, des oxydes de métaux, des hydroxydes de métaux, des céramiques, des composites granulaires. Contrairement aux matériaux de l'art antérieur, le matériau obtenu par le procédé de l'invention ne voit pas sa structure ni sa composition chimique modifiées par le procédé de consolidation.

Le monolithe poreux obtenu par le procédé de l'invention est constitué essentiellement d'un matériau choisi parmi les métaux, les dérivés de métaux, les oxydes de métaux, les hydroxydes de métaux, les céramiques, les composites et leurs mélanges. Un tel matériau peut éventuellement comprendre un ou plusieurs composants organiques tels que des principes actifs thérapeutiques, des agents de dopage. Contrairement aux monolithes poreux de l'art antérieur, le matériau obtenu par le procédé de l'invention ne requiert pas l'addition de matériaux gélifiants ou de polymères épaississants ou viscosifiants pour présenter une tenue mécanique satisfaisante.

Avantageusement les métaux, les dérivés de métaux, les oxydes de métaux, les hydroxydes de métaux, les céramiques, les composites représentent au moins 90% en masse, de préférence au moins 95 %, mieux au moins 98% en masse par rapport à la masse totale du monolithe poreux.

Avantageusement, les matériaux gélifiants ou les polymères épaississants ou viscosifiants représentent moins de 10% en masse, encore mieux moins de 5% et de préférence moins de 2% en masse par rapport à la masse totale du monolithe poreux.

Le monolithe poreux obtenu par le procédé de l'invention est en particulier à base d'hydroxyapatite dont la structure chimique est celle de l'hydroxyapatite granulaire, soit Ca₁₀(PO4)₆(OH)₂.

Le monolithe poreux obtenu par le procédé de l'invention est en particulier à base de BCP, dont la structure chimique est celle du BCP granulaire, soit un mélange de HA : Ca₁₀(PO4)₆(OH)₂ et de β-TCP : Ca₃(PO₄)₂.

Le monolithe poreux obtenu par le procédé de l'invention est en particulier à base d'alumine, dont la structure chimique est celle de l'alumine granulaire, soit Al₂O₃.

Le monolithe poreux obtenu par le procédé de l'invention est en particulier à base de zircone, dont la structure chimique est celle de la zircone granulaire, soit ZrO₂.

Le monolithe poreux obtenu par le procédé de l'invention est en particulier à base de TiO₂, dont la structure chimique est celle du dioxyde de titane granulaire anatase.

### Utilisations des matériaux monolithiques poreux :

Les matériaux monolithiques trouvent des applications dans de nombreux domaines :
- comme matériau de substitution osseuse ou dentaire, en tant que prothèse rigide et poreuse, pour la régénération osseuse ou dentaire, dans le cas des matériaux à base d'hydroxyapatite,
- pour les applications dans des plaques anodiques, dispositifs nanoélectroniques, périphériques de stockage haute densité, capteurs chimiques sensibles et pour les nanomatériaux fonctionnels dans le cas de l'alumine poreuse;
- pour les applications dans des piles à combustible d'oxyde, les filtres céramiques, les barrières thermiques, les applications dentaires et les matériaux biologiques dans le cas de la Zircone poreuse ;
- pour applications catalytiques photoniques dans le cas de l'oxyde de titane poreux ;
- pour obtenir de fine couches d'oxyde de titane poreux avec des biocéramiques microporeuses sur métaux / céramiques biocompatibles tels que Al₂O₃, ZrO₂ et Ti avec ou sans délivrance de molécules thérapeutiques pour une guérison locale ou pour reprise cellulaire de l'os accélérée.

### Partie expérimentale :

### I-Matériaux et méthodes

- Hydroxyapatite : on a utilisé une hydroxyapatite (formule Ca₁₀(PO4)₆(OH)₂) de pureté 99.99% de granulométrie 15-25 nm, commercialisée par la société American Elements sous le nom Hydroxyapatite nanopowders.
- Gélatine : on a utilisé une gélatine commercialisée par la société Sigma Aldrich sous le nom Gelatin Solution type B, 2%.
- Collagène : on a utilisé un collagène commercialisé par la société Sigma Aldrich sous le nom de Collagen, type I solution from rat tail Bioreagent.
- Agent de réticulation : on a utilisé le Glutaraldéhyde solution Grade I, 25 % dans H₂O commercialisé par la société Sigma Aldrich.
- Alumine : on a utilisé une alumine (formule Al₂O₃) de pureté 99% de granulométrie 20 nm commercialisée par la société US-Nano sous le nom Al₂O₃.
- Dioxyde de titane : on a utilisé un dioxyde de titane (formule TiO₂) de pureté 99,7% de granulométrie 25 -100 nm de qualité anatase ou rutile, commercialisée par la société Sigma Aldrich
- Dioxyde de zirconium : On a utilisé une zircone (formule ZrO₂) de pureté 99,9999%, de granulométrie 20-40 nm de structure (3YSZ) ou (8YSZ)
- Tube : le tube flexible est en silicone d'épaisseur 1mm, il est commercialisé par la société MacoPharma.
- Presse isostatique : constituée d'une partie « Haute Pression » : enceinte HP (ICMCB) contenant l'échantillon et l'huile silicone (fluide transmetteur de pression) et d'une pompe hydropneumatique (NovaSwiss) permettant de comprimer le fluide et d'une seconde partie « Basse Température » : cuve dans laquelle est placée l'enceinte HP contenant l'huile silicone réfrigérée avec un cryoplongeur (Huber)
- Résistance mécanique : la tenue mécanique est évaluée en première approche par une observation de tenue manuelle. On considère que la résistance mécanique est satisfaisante si le matériau peut être manipulé sans dégradation.
- Etude de la porosité : la porosité est mesurée par la microscopie électronique à balayage (MEB).

### II- Exemples

### II.1. Préparation de la suspension :

Des suspensions de produit sont préparées avec les composants et les proportions décrits dans les tableaux 1.1 à 1.4 ci-dessous.

1g de matériau minéral est introduit dans un mélangeur en présence éventuellement d'additifs et avec différentes quantités d'eau en fonction de la quantité de porosité requise, pour préparer une suspension en mélangeant de façon homogène.

On indique dans les tableaux 1.1 à 1.4 les pourcentages massiques (Wt %) de chaque matériau.

Dans le cas des tableaux 1.2 et 1.3 on a procédé en deux étapes : Dans une première étape on réalise le mélange HAp+Additif en mettant en oeuvre HAp et l'additif (gélatine ou collagène) dans les ratios donnés dans les colonnes correspondantes. Dans une seconde étape on additionne à ce premier mélange la quantité d'eau indiquée dans la colonne de droite de façon à atteindre un total de 100%.

### Exemple 1.1: Hydroxyapatite (HAp) pur poreux

**Tableau 1.1**

| | HAp Wt% | Eau Wt% |
|---|---|---|
| Exemple 1.1.a | 90 | 10 |
| Exemple 1.1.b | 80 | 20 |
| Exemple 1.1.c | 70 | 30 |
| Exemple 1.1.d | 60 | 40 |

### Exemple 1.2: Hydroxyapatite (HAp) poreux avec gélatine

**Tableau 1.2**

| | Pré-mélange | | Eau Wt% |
|---|---|---|---|
| | HAp Wt% | Additif Wt% | |
| Exemple 1.2.a | 65 | Gélatine 35 % | 50 |
| Exemple 1.2.b | 65 | Gélatine 35 % | 70 |
| Exemple 1.2.c | 65 | Gélatine 35 % | 90 |
| Exemple 1.2.d | 85 | Gélatine 15% | 50 |
| Exemple 1.2.e | 85 | Gélatine 15% | 70 |
| Exemple 1.2.f | 85 | Gélatine 15% | 90 |

### Exemple 1.3: Hydroxyapatite (HAp) poreux avec collagène

**Tableau 1.3**

| | Pré-mélange | | Eau Wt% |
|---|---|---|---|
| | HAp Wt% | Additif Wt% | |
| Exemple 1.3.a | 65 | Collagène 35 % | 50 |
| Exemple 1.3.b | 65 | Collagène 35 % | 70 |
| Exemple 1.3.c | 65 | Collagène 35 % | 90 |
| Exemple 1.3.d | 85 | Collagène 15% | 50 |
| Exemple 1.3.e | 85 | Collagène 15% | 70 |
| Exemple 1.3.f | 85 | Collagène 15% | 90 |

### Exemple 1.4 : Matériaux variés

**Tableau 1.4**

| | Matériau | % matériau | Eau Wt% |
|---|---|---|---|
| Exemple 1.4.a | HAp | 80 wt% HAp | 20 |
| Exemple 1.4.b | Al₂O₃ | 60 wt% Al₂O₃ | 40 |
| Exemple 1.4.c | TiO₂ | 70 wt % TiO₂ | 30 |
| Exemple 1.4.d | 8YSZ | 70 wt % 8YSZ | 30 |
| Exemple 1.4.e | 3YSZ | 70 wt % 3YSZ | 30 |
| Exemple 1.4.f | ZrO₂ | 70 wt % ZrO₂ | 30 |

### II.2 Cycle de traitement :

La suspension de 1g est transférée dans un tube flexible résistant à la température négative et mis dans la presse isostatique à T≈ -6°C à -20°C. La glace est formée dans la suspension qui est soumise à diverses pressions (100MPa - 400MPa) pour différents intervalles de temps pour obtenir un échantillon dur. La résistance mécanique de l'échantillon est variée en fonction du temps de palier et le cycle de pression (pression du cycle de l'étape unique / à plusieurs étapes). L'échantillon après compression est ensuite lyophilisé pour transformer l'eau de l'état solide à l'état gazeux. Ensuite, l'échantillon est sorti de la poche ; il présente une structure poreuse et une tenue mécanique élevée. De façon optionnelle, l'échantillon est plongé dans une solution d'agent de réticulation.

Les cycles de traitement auxquels sont soumises les suspensions sont décrits de façon détaillée dans les tableaux ci-dessous.
- Cycle A

**Tableau A**

| | Température | Pression | Durée |
|---|---|---|---|
| Etape 1 | -15 °C, - 5°C | 1MPa | 10 Min, 20 Min |
| Etape 2 | -15 °C, - 5°C | 100, 200, 300 MPa | 5 Min, 10 Min, 15 Min |
| Etape 3 | -15 °C, - 5°C | 100, 200, 300 MPa | 5 Min, 10 Min, 15 Min |
| Etape 4 | -15 °C, - 5°C | 1 MPa | 5 Min |
| Etape 5 | 0°C | 0 MPa | 1 Min |
| Etape 6 (lyophilisation) | Ambiante | 0 MPa | 2 h |

- Cycle B

**Tableau B**

| | Température | Pression | Durée | Trempage dans un agent réticulant |
|---|---|---|---|---|
| Etape 1 | -15 °C, - 5°C | 1 MPa | 10 Min, 20 Min | - |
| Etape 2 | -15 °C, - 5°C | 100, 200, 300 MPa | 5 Min, 10 Min, 15 Min | - |
| Etape 3 | -15 °C, - 5°C | 100, 200, 300 MPa | 5 Min, 10 Min, 15 Min | - |
| Etape 4 | -15 °C, - 5°C | 1 MPa | 5 Min | - |
| Etape 5 | 0°C | 0 MPa | 1 Min | - |
| Etape 6 (lyophilisation) | Ambiante | 0 MPa | 2 h | - |
| Etape 7 | Ambiante | 0 MPa | 1 Min | Oui |

### II.3 Résultats :

Les matériaux suivants ont été préparés en partant des suspensions décrites ci-dessus et en appliquant les cycles de traitement décrits ci-dessus, les conditions de traitement de chaque échantillon sont résumées dans les tableaux 3.1 à 3.4, ainsi que les propriétés des produits obtenus :

### Exemple 3.1: Hydroxyapatite (HAp) pur poreux

**Tableau 3.1**

| Suspension | Cycle | Observation | Porosité |
|---|---|---|---|
| Exemple 1.1.a | A | rigide | ≤ 65 % |
| Exemple 1.1.b | A | rigide | ≤ 55 % |
| Exemple 1.1.c | A | rigide | ≤ 45 %, |
| Exemple 1.1.d | A | rigide | ≤ 35 % |

### Exemple 3.2: Hydroxyapatite (HAp) poreux avec gélatine

**Tableau 3.2**

| Suspension | Cycle | Observation | Porosité |
|---|---|---|---|
| Exemple 1.2.a | B | rigide | ≤ 35 % |
| Exemple 1.2.b | B | rigide | ≤ 45 % |
| Exemple 1.2.c | B | rigide | ≤ 55 % |
| Exemple 1.2.d | B | rigide | ≤ 35 % |
| Exemple 1.2.e | B | rigide | ≤ 45 % |
| Exemple 1.2.f | B | rigide | ≤ 55 % |

### Exemple 3.3: Hydroxyapatite (HAp) poreux avec collagène

**Tableau 3.3**

| Suspension | Cycle | Observation | Porosité |
|---|---|---|---|
| Exemple 1.3.a | B | rigide | ≤ 35 % |
| Exemple 1.3.b | B | rigide | ≤ 45 % |
| Exemple 1.3.c | B | rigide | ≤ 55 % |
| Exemple 1.3.d | B | rigide | ≤ 35 % |
| Exemple 1.3.e | B | rigide | ≤ 45 % |
| Exemple 1.3.f | B | rigide | ≤ 55 % |

### Exemple 3.4 : Matériaux variés

**Tableau 3.4**

| Suspension | Cycle | Observation | Porosité |
|---|---|---|---|
| Exemple 1.4.a | A | Rigide | 55% |
| Exemple 1.4.b | A | Rigide | 65% |
| Exemple 1.4.c | A | Rigide | 65% |
| Exemple 1.4.d | A | Rigide | 35% |
| Exemple 1.4.e | A | Rigide | 30% |
| Exemple 1.4.f | A | Rigide | 20% |

La structure des matériaux a été observée au moyen d'un appareil Scios Dual Beam, FEI, France. La microscopie FIB-SEM et la tomographie électronique STEM-EDS ont été réalisées sur l'échantillon de l'exemple 1.1 b. Les observations ont été réalisées de façon à permettre la quantification des distributions spatiales morphologiques et géométriques du réseau poreux multi-échelles à des échelles de longueur allant de quelques dizaines de microns à moins d'un nanomètre. L'analyse de la microstructure a été effectuée sous vide élevé à une tension de 2 kV. Les clichés montrent une répartition homogène de la porosité dans le monolithe fabriqué par le procédé de l'invention.

## Revendications

1. Procédé de fabrication d'un matériau monolithique poreux (5), ce procédé comprenant au moins les étapes suivantes :
(a) Fourniture d'au moins une poudre minérale ou organique (S),
(b) Préparation d'un mélange (1) comprenant au moins la poudre de l'étape (a) et au moins un solvant, de préférence de l'eau,
(c) Conditionnement du mélange de l'étape (b) dans un emballage étanche (2) en matériau élastiquement déformable pour former un échantillon (3),
(d) Application à l'échantillon (3) de l'étape (c) d'une pression (P) supérieure ou égale à 50MPa à une température où le solvant est sous forme solide,
(e) Retour à la pression atmosphérique,
(f) Ouverture de l'emballage et récupération du matériau,
(g) Elimination du solvant à une température où le solvant est sous forme solide.

2. Procédé selon la revendication 1 dans lequel la poudre (S) est choisie parmi les poudres minérales ou inorganiques.

3. Procédé selon la revendication 2, **caractérisé en ce que** les poudres minérales ou inorganiques sont choisies parmi les métaux, les céramiques et les matériaux composites.

4. Procédé selon la revendication 3, **caractérisé en ce que** les métaux sont choisis parmi les métaux alcalins et alcalino-terreux, les métaux de transition, leurs sels et leurs dérivés.

5. Procédé selon la revendication 4, **caractérisé en ce que** les sels et les dérivés sont choisis parmi les oxydes, les hydroxydes, les sels de phosphate, de carbonate, les borures, les carbures et les nitrures.

6. Procédé selon la revendication 1 ou la revendication 2 dans lequel la poudre (S) est minérale ou inorganique et est choisie parmi : l'hydroxyapatite, l'alumine, le dioxyde de titane, la zircone, la zircone stabilisée à l'Yttrium (YSZ), la zircone stabilisée à la Magnésie (PSZ).

7. Procédé selon l'une quelconque des revendications précédentes dans lequel le mélange (1) de l'étape (b) comprend au moins un composant organique.

8. Procédé selon la revendication 7, **caractérisé en ce que** le composant organique est choisi parmi : un principe actif thérapeutique ; des peptides ; des protéines ; des agents coagulants ; des marqueurs ; des facteurs de croissance ; des polymères naturels ou de synthèse ; des agents de dopage pour l'électronique ; des polymères électroconducteurs ; des capteurs chimiques ; un agent de réticulation ; un solvant miscible à l'eau.

9. Procédé selon l'une quelconque des revendications précédentes dans lequel le mélange (1) comprend de 50 à 95 % en masse de poudre (S), par rapport à la masse totale du mélange.

10. Procédé selon l'une quelconque des revendications précédentes dans lequel l'étape (d) comprend au moins les sous-étapes suivantes :
(d1) Un conditionnement de l'échantillon à pression atmosphérique et à une température choisie pour que le solvant, de préférence l'eau, soit sous forme solide,
(d2) L'application d'une pression supérieure ou égale à 50MPa à une température choisie pour que le solvant, de préférence l'eau, soit sous forme solide.

11. Procédé selon l'une quelconque des revendications précédentes dans lequel l'étape (d) comprend au moins un conditionnement à une température allant de -5°C à -60°C et à une pression allant de 50MPa à 700MPa.

12. Procédé selon l'une quelconque des revendications précédentes dans lequel le solvant est de l'eau et l'élimination de l'eau à l'étape (g) est réalisée par lyophilisation.

## Patentansprüche

1. Verfahren zur Herstellung eines porösen monolithischen Materials (5), wobei das Verfahren mindestens die folgenden Schritte umfasst:
(a) Bereitstellen mindestens eines anorganischen oder organischen Pulvers (S),
(b) Zubereiten einer Mischung (1), die mindestens das Pulver aus Schritt (a) und mindestens ein Lösungsmittel, vorzugsweise Wasser, enthält,
(c) Konditionieren der Mischung aus Schritt (b) in einer geschlossenen Umhüllung (2) aus elastisch verformbarem Material zur Bildung einer Probe (3),
(d) Beaufschlagen der Probe (3) aus Schritt (c) mit einem Druck (P) größer oder gleich 50 MPa bei einer Temperatur, bei der das Lösungsmittel in fester Form vorliegt,
(e) Wiederherstellen des atmosphärischen Drucks,
(f) Öffnen der Umhüllung und Rückgewinnung des Materials,
(g) Entfernen des Lösungsmittels bei einer Temperatur, bei der das Lösungsmittel in fester Form vorliegt.

2. Verfahren nach Anspruch 1, wobei das Pulver (S) aus mineralischen oder anorganischen Pulvern ausgewählt ist.

3. Verfahren nach Anspruch 2, wobei die mineralischen oder anorganischen Pulver aus Metallen, Keramiken und Verbundwerkstoffen ausgewählt sind.

4. Verfahren nach Anspruch 3, **dadurch gekennzeichnet, dass** die Metalle aus Alkali- und Erdalkalimetallen, Übergangsmetallen, deren Salzen und Derivaten ausgewählt sind.

5. Verfahren nach Anspruch 4, **dadurch gekennzeichnet, dass** die Salze und Derivate ausgewählt sind aus Oxiden, Hydroxiden, Phosphatsalzen, Carbonaten, Boriden, Carbiden und Nitriden.

6. Verfahren nach Anspruch 1 oder Anspruch 2, wobei das Pulver (S) mineralisch oder anorganisch ist und ausgewählt ist aus: Hydroxylapatit, Aluminiumoxid, Titandioxid, Zirkonoxid, Yttriumoxid-stabilisiertes Zirkonoxid (YSZ), Magnesiumoxid-stabilisiertes Zirkonoxid (PSZ).

7. Verfahren nach einem der vorangehenden Ansprüche, wobei die Mischung (1) von Schritt (b) mindestens eine organische Komponente enthält.

8. Verfahren nach Anspruch 7, **dadurch gekennzeichnet, dass** die organische Komponente ausgewählt ist aus: einem therapeutischen Wirkstoff; Peptiden; Proteinen; Gerinnungsmitteln; Markern; Wachstumsfaktoren; natürlichen oder synthetischen Polymeren; Dotiermitteln für die Elektronik; elektrisch leitfähigen Polymeren; chemischen Sensoren; einem Vernetzungsmittel; einem mit Wasser mischbaren Lösungsmittel.

9. Verfahren nach einem der vorangehenden Ansprüche, wobei die Mischung (1) 50 bis 95 Gew.-% Pulver (S), bezogen auf das Gesamtgewicht der Mischung, enthält.

10. Verfahren nach einem der vorangehenden Ansprüche, wobei Schritt (d) mindestens die folgenden Teilschritte umfasst:
(d1) Konditionierung der Probe bei Atmosphärendruck und bei einer Temperatur, die so gewählt ist, dass das Lösungsmittel, vorzugsweise Wasser, in fester Form vorliegt,
(d2) Anlegen eines Drucks von 50 MPa oder mehr bei einer Temperatur, die so gewählt ist, dass das Lösungsmittel, vorzugsweise Wasser, in fester Form vorliegt.

11. Verfahren nach einem der vorangehenden Ansprüche, wobei Schritt (d) mindestens eine Konditionierung bei einer Temperatur im Bereich von -5°C bis -60°C und bei einem Druck im Bereich von 50 MPa bis 700 MPa umfasst.

12. Verfahren nach einem der vorangehenden Ansprüche, wobei das Lösungsmittel Wasser ist und das Entfernen des Wassers in Schritt (g) durch Gefriertrocknung durchgeführt wird.

## Claims

1. Method for producing a porous monolithic material (5), this method comprising at least the following steps:
(a) Supplying at least one mineral or organic powder (S),
(b) Preparing a mixture (1) comprising at least the powder of step (a) and at least one solvent, preferably water,
(c) Packing the mixture of step (b) in sealed packaging (2) made from an elastically deformable material in order to form a sample (3),
(d) Applying to the sample (3) of step (c) a pressure (P) greater than or equal to 50 MPa at a temperature at which the solvent is in solid form,
(e) Returning to atmospheric pressure,
(f) Opening the packaging and recovering the material,
(g) Removing the solvent at a temperature at which the solvent is in solid form.

2. Method according to claim 1, in which the powder (S) is chosen among mineral or organic powders.

3. Method according to claim 2, in which the mineral or organic powders are chosen among metals, ceramics and composite materials.

4. Method according to claim 3, in which the metals are chosen among alkali and alkaline earth metals, transition metals, their salts and derivatives.

5. Method according to claim 4, in which the salts and derivatives are chosen among oxides, hydroxides, salts of phosphate, of carbonate, borides, carbides and nitrides.

6. Method according to claim 1 or to claim 2; in which the powder (S) is mineral or organic and is chosen among: hydroxyapatite, alumina, titanium dioxide, zirconia, Yttria-stabilized zirconia (YSZ) Magnesia-stabilized zirconia (PSZ).

7. Method according to any one of the preceding claims, in which the mixture (1) of step (b) comprises at least one organic component.

8. Method according to claim 7, in which the organic component is selected from: a therapeutic active ingredient; peptides; proteins; coagulants; markers; growth factors; natural or synthetic polymers; dopants for electronics; electrically conductive polymers; chemical sensors; a cross-linking agent; a water-miscible solvent.

9. Method according to any one of the preceding claims, in which the mixture (1) comprises 50 to 95% by weight of powder (S), with respect to the total weight of the mixture.

10. Method according to any one of the preceding claims, in which step (d) comprises at least the following sub-steps:
(d1) Conditioning the sample at atmospheric pressure and at a temperature selected so that the solvent, preferably water, is in solid form,
(d2) Applying a pressure greater than or equal to 50 MPa at a temperature selected so that the solvent, preferably water, is in solid form.

11. Method according to any one of the preceding claims, in which step (d) comprises at least conditioning at a temperature of between -5°C and -60°C and at a pressure of between 50 MPa and 700 MPa.

12. Method according to any one of the preceding claims, in which the solvent is water and the removal of the water in step (g) is carried out by lyophilisation.
